(19) Europäisches Patentamt
European Patent Office
Office européen des brevets

(11) EP 0 755 676 B2

(12) **NOUVEAU FASCICULE DE BREVET EUROPEEN**

(45) Date de publication et mention de la décision concernant l'opposition:
**12.12.2001 Bulletin 2001/50**

(45) Mention de la délivrance du brevet:
**01.04.1998 Bulletin 1998/14**

(21) Numéro de dépôt: **96401508.5**

(22) Date de dépôt: **09.07.1996**

(51) Int Cl.⁷: $A61K\ 7/48$, $A61K\ 7/06$, $A61K\ 7/04$, $A61K\ 7/043$

(54) **Utilisation d'acides monocarboxyliques pour le traitement des matières kératiniques**

Verwendung von Monocarbonsäuren zur Pflege von Keratinsubstanzen

Use of monocarboxylic acids for the care of keratineous matters

(84) Etats contractants désignés:
**DE ES FR GB IT**

(30) Priorité: **28.07.1995 FR 9509251**

(43) Date de publication de la demande:
**29.01.1997 Bulletin 1997/05**

(73) Titulaire: **L'OREAL**
**75008 Paris (FR)**

(72) Inventeurs:
• **Ramin, Roland**
**91760 Itteville (FR)**
• **Garson, Jean-Claude**
**92150 Suresnes (FR)**

(74) Mandataire: **Dodin, Catherine**
**L'OREAL-DPI**
**6 rue Bertrand Sincholle**
**92585 Clichy Cédex (FR)**

(56) Documents cités:
EP-A- 0 102 534  WO-A-85/00288
WO-A-88/08701  US-A- 2 729 586

• **K. Schrager, Grundlagen und Rezepturen der Kosmetika, Dr. A. Hüthig Verlag, Heidelberg, 1979, pages 477, 610, 611**
• **K. Schrader, Grundlagen und Rezepturen der Kosmetika, 2. Edition, Dr. A. Hüthig Verlag, Heidelberg, pages 730, 732, 737, 766, 863**
• **J.S. Jellinek, Josmetologie, 3. edition, 1979, pages 366, 367**
• **M.G. de Navarre, The Chemistry and Manufacture of Cosmetics, page 113**
• **The Merck Index, edition 1989, pages 363, 709, 842, 1375, 1433**

EP 0 755 676 B2

**Description**

La présente invention a pour objet l'utilisation d'acides carboxyliques dans des compositions cosmétiques, pour le traitement des ongles.

Il est bien connu que les ongles présentent, de façon fréquente, des défauts de structure et de consistance, ceux-ci pouvant être d'origine diverses et notamment liés au fonctionnement interne de l'individu, à ses conditions de vie, à son mode d'alimentation, à son âge, à ses états de fatigue ou de surmenage.

Ces défauts peuvent également apparaître sous l'effet d'actions qui érodent, à la suite par exemple d'expositions prolongées ou répétées à des agents détergents, à des solvants, à des produits chimiques en particulier ménager, à des atmosphères chaudes ou froides, humides ou sèches, ou à des expositions aux rayonnements UV.

Ces défauts de structure et de consistance ont pour effet de rendre la surface des ongles inesthétique, ce qui peut être source de gêne et de désagréments multiples.

En vue de renforcer les ongles, on a déjà proposé différents types de composition essentiellement basée sur l'emploi soit d'agents réticulants des protéines destinées à renforcer le réseau.kératinique comme par exemple le formol (voir Forslind B., Acta Dermatovener, vol 60, p 217-222 1980), soit d'agents à fonction essentiellement nutritive tels que par exemple la cystine, le cholestérol, la S-carboxyméthylcystéïne ou encore des extraits de collagène.

L'utilisation de tels agents réticulants ou de tels agents à fonction nutritive né permet pas cependant d'obtenir de bons résultats et présente par ailleurs certains inconvénients. En particulier, les produits à base de formol peuvent provoquer certaines réactions d'allergie.

La demanderesse a découvert qu'en utilisant certains acides monocarboxyliques, il était possible d'obtenir un remarquable effet de durcissement des ongles, tout en évitant les risques d'intolérance et de sensibilisation.

La présente invention a donc pour objet l'utilisation selon la revendication 1.

De préférence, on peut utiliser les acides monocarboxyliques ayant de 2 à 5 atomes de carbone. Les acides monocarboxyliques selon l'invention peuvent être linéaires ou ramifiés. Ils peuvent être choisis parmi l'acide acétique, l'acide propionique, l'acide butyrique, l'acide valérique, l'acide caproïque, l'acide heptanoïque ou l'acide caprylique. On utilise de préférence l'acide acétique.

L'acide monocarboxylique utilisé selon l'invention peut être un produit de synthèse. Il peut également être d'origine naturelle.

Par ailleurs, l'acide monocarboxylique utilisé selon l'invention peut se présenter dans la composition finale sous forme d'acide libre et/ou sous la forme de l'un de ses sels associés (sels avec une base organique ou un alcalin notamment).

Selon l'invention, la teneur en acide monocarboxylique peut aller de 0,01 % à 20 % en poids, et de préférence de 0,5 % à 3 %, par rapport au poids total de la composition.

Par ailleurs, la composition cosmétique de l'invention comprend un support cosmétiquement acceptable. Ce support peut comprendre des solvants organiques, de l'eau, et/ou est un milieu huileux, par exemple.

Comme solvants organiques, on peut citer les cétones, comme l'acétone, la méthyl-éthyl-cétone, la méthyl-isobutyl-cétone; les éthers de glycols; les alcools comme l'éthanol, le n-butanol, le n-propanol, l'isopropanol; les acétates comme l'acétate de butyle, d'éthyle, d'isopropyle, l'acétate de méthoxy-2-éthyle; les hydrocarbures linéaires ou ramifiés tels que l'hexane ou l'octane; ou encore les hydrocarbures aromatiques tels que le xylène et le toluène.

Lorsque le support cosmétiquement acceptable comprend de l'eau, la composition peut se présenter notamment sous la forme d'une solution aqueuse ou hydroalcoolique, d'une émulsion huile-dans-eau ou eau-dans-huile, voire d'une émulsion multiple, ou encore sous la forme d'un gel aqueux.

Ledit milieu huileux peut comprendre une ou plusieurs huiles, volatiles et/ou non volatiles, par exemple d'origine végétale, minérale, animale et/ou de synthèse, parmi lesquelles on peut citer:

.   les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat, les huiles de poisson, le tricaprocaprylate de glycérol, ou les huiles végétales ou animales de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin ;

.   les huiles essentielles naturelles ou synthétiques telles que, par exemple, les huiles d'eucalyptus, de lavandin, de lavande, de vétiver, de citron, de santal, de romarin, de camomille, de sarriette, de noix de muscade, de cannelle, d'hysope, de carvi, d'orange, de géraniol, de cade et de bergamote;

.   les hydrocarbures, tels que l'hexadécane et l'huile de paraffine ;

.   les esters d'acide minéral et d'un alcool ;

.   les éthers et les polyéthers ;

.   les huiles et gommes de silicones.

De plus, la composition peut comprendre un polymère filmogène, ce qui permet de déposer sur l'ongle, un film résistant qui assure un contact prolongé de l'acide monocarboxylique avec la surface de l'ongle. A titre d'exemple, le polymère peut être choisi parmi la nitrocellulose, l'acétobutyrate de cellulose, les butyralpolyvinyliques, les résines alkydes, les polyesters, les acryliques, les polyuréthannes.

Les polymères peuvent être dissous ou dispersés dans la composition. Ils peuvent être généralement présents à une teneur allant de 1 % à 40 % en poids par rapport au poids total de la composition.

La composition pour l'utilisation selon l'invention peut également comprendre, en plus du polymère filmogène, des plastifiants qui permettent de régler la flexibilité du film sans affaiblir sa résistance physique.

Les plastifiants utilisables sont ceux couramment employés dans les compositions de vernis à ongles. Comme plastifiants, on peut citer les phtalates de dibutyle, de dioctyle, de di-isobutyle, de diméthoxyéthyl, les benzoates de benzyle, de glycéryle; les citrates de triéthyle, de tributyle, l'acétyl-citrate de tributyle; les phosphates de tributyle, de triphényle; les glycols; le camphre ainsi que leurs dérivés et leurs mélanges. Les plastifiants peuvent être généralement présents à une teneur allant de 1 % à 30 % en poids par rapport au poids total de la composition.

Par ailleurs, la composition pour l'utilisation selon l'invention peut contenir des adjuvants couramment utilisés dans les compositions cosmétiques. On peut citer à titre d'exemple d'adjuvants les colorants, les pigments, les nacres, les laques, les agents anti-UV, les agents épaississants, les tensioactifs, les cires, les parfums, et des actifs de tels que le D-panthénol, le phytantriol, les vitamines et leurs dérivés, la kératine et ses dérivés, la mélanine, le collagène, la cystine, le chitosane et ses dérivés, les céramides, la biotine, les oligo-éléments, la glycérine, les hydrolysats de protéines, les phospholipides, les agents hydratants. Bien entendu, l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

La composition pour l'utilisation selon l'invention peut se présenter sous forme de composition de vernis à ongles ou de soin des ongles.

On va maintenant donner des exemples illustrant la présente invention sans toutefois la limiter.

### Exemple 1 :

On a déterminé la dureté d'un ongle traité par l'acide acétique.

### Principe :

On applique sur l'ongle un pénétromètre en forme de pyramide à base carrée, à l'aide d'une charge P. On détermine ensuite la dimension moyenne d'une diagonale de l'empreinte carrée obtenue avec le pénétromètre.
La dureté VICKERS (HV) est alors déterminée par la relation :

$$HV = \frac{1854,4 \times P}{d^2}$$

d = diagonale moyenne en $\mu$m
P = charge appliquée en g

La mesure de la dureté VICKERS est effectuée à l'aide du microduromètre M 400 g 2 de la société LECO.

### Protocole :

On immerge des fragments d'ongles pendant 3 heures dans de l'eau distillée, puis on laisse à l'humidité ambiante pendant 24 heures.
On applique ensuite 2 $\mu$l de solution aqueuse d'acide acétique à 10 % en poids sur l'ongle. Les ongles sont ensuite placés 3 jours dans une atmosphère d'humidité relative à 75 %. On effectue alors la mesure de la dureté VICKERS.
On effectue une deuxième application du produit et une deuxième mesure de dureté selon les mêmes conditions précédemment décrites.

Le test est conduit sur trois échantillons, comparativement à de l'eau (placebo).

### Résultats :

On a obtenu les résultats suivants :

| Traitement | Dureté initiale sans traitement | Dureté après la première application | Dureté après la deuxième application |
|---|---|---|---|
| Eau | $11,4 \pm 0,6$ | $10,7 \pm 0,7$ | $109 \pm 0,2$ |
| Acide acétique | $10,7 \pm 0,7$ | $12,5 \pm 0,4$ (+ 16,8 %) | $11,9 \pm 0,8$ (+11,2%) |

Les valeurs rapportées correspondent à la moyenne obtenue pour trois échantillons. Les chiffres indiqués entre parenthèse indiquent l'augmentation de la dureté de l'ongle traité par rapport à l'ongle avant traitement.

On constate que la dureté des ongles, après traitement par l'acide acétique, augmente sensiblement.

Ces essais confirment que l'acide acétique a la propriété de durcir l'ongle.

**Exemple 2 :**

On a préparé une base incolore ayant la composition suivante :

| - nitrocellulose | | 15 g |
|---|---|---|
| - plastifiant et résine | | 15 g |
| - alcool isopropylique | | 9 g |
| - acide acétique | | 1 g |
| - solvant (acétate d'éthyle, acétate de butyle) | qsp | 100 g |

Après application de la composition sur l'ongle et après séchage, on obtient un film lisse et homogène. On applique cette composition pendant 8 semaines sur des ongles durs, tous les 3 jours. avant chaque application, l'ancien film est retiré des ongles à l'aide d'un dissolvant classique. On constate que les ongles ainsi traités sont durcis.

## Revendications

1.   Utilisation, dans une composition cosmétique à appliquer sur l'ongle, d'au moins un acide monocarboxylique ayant de 2 à 8 atomes de carbone, et/ou d'un de ses sels, comme agent durcisseur des ongles, ledit acide ne comportant pas de groupement hydroxyle.

2.   Utilisation selon la revendication 1, **caractérisée par le fait que** l'acide monocarboxylique a de 2 à 5 atomes de carbone.

3.   Utilisation selon l'une quelconque des revendications précédentes, **caractérisée par le fait que** l'acide monocarboxylique est sous forme acide libre.

4.   Utilisation selon l'une des revendications précédentes, **caractérisée par le fait que** l'acide monocarboxylique est choisi parmi l'acide acétique, l'acide propionique, l'acide butyrique, l'acide valérique, l'acide caproïque, l'acide heptanoïque ou l'acide caprylique.

5.   Utilisation selon l'une des revendications précédentes, **caractérisée par le fait que** l'acide monocarboxylique est l'acide acétique.

6.   Utilisation selon l'une quelconque des revendications précédentes, **caractérisée par le fait que** la composition comprend une teneur en acide monocarboxylique de 0,01 % à 20 % en poids.

7.   Utilisation selon l'une quelconque des revendications précédentes, **caractérisée par le fait que** la composition comprend une teneur en acide monocarboxylique de 0,5 % à 3 % en poids.

8.   Utilisation selon l'une quelconque des revendications précédentes, **caractérisée par le fait que** la composition comprend un support cosmétiquement acceptable choisi parmi les solvants organiques, l'eau et/ou les huiles.

9.   Utilisation selon l'une quelconque des revendications précédentes, **caractérisée par le fait que** la composition comprend un polymère filmogène choisi parmi la nitrocellulose, l'acétobutyrate de cellulose, les butyralpolyvinyliques, les résines alkydes, les polyesters, les acryliques, les polyuréthannes.

10. Utilisation selon la revendication 9, **caractérisée par le fait que** la composition comprend un agent plastifiant.

11. Utilisation selon l'une quelconque des revendications précédentes, **caractérisée par le fait que** la composition est un vernis à ongles ou une composition de soin des ongles.

**Patentansprüche**

1. Verwendung mindestens einer Monocarbonsäure mit 2 bis 8 Kohlenstoffatomen und/oder eines Salzes dieser Monocarbonsäure als Mittel zur Härtung von Nägeln in einer kosmetischen Zusammensetzung, die auf diese Nägel aufgetragen werden soll, wobei die Säure keine Hydroxygruppen enthält.

2. Verwendung nach Anspruch 1, **dadurch gekennzeichnet, daß** die Monocarbonsäure 2 bis 5 Kohlenstoffatome aufweist.

3. Verwendung nach einem der vorhergehenden Ansprüche, **dadurch gekennzeichnet, daß** die Monocarbonsäure in Form der freien Säure vorliegt.

4. Verwendung nach einem der vorhergehenden Ansprüche, **dadurch gekennzeichnet, daß** die Monocarbonsäure unter Essigsäure, Propionsäure, Buttersäure, Valeriansäure, Capronsäure, Oenanthsäure oder Caprylsäure ausgewählt ist.

5. Verwendung nach einem der vorhergehenden Ansprüche, **dadurch gekennzeichnet, daß** die Monocarbonsäure die Essigsäure ist.

6. Verwendung nach einem der vorhergehenden Ansprüche, **dadurch gekennzeichnet, daß** die Zusammensetzung einen Monocarbonsäureanteil von 0,01 bis 20 Gew.-% enthält.

7. Verwendung nach einem der vorhergehenden Ansprüche, **dadurch gekennzeichnet, daß** die Zusammensetzung einen Monocarbonsäureanteil von 0,5 bis 3 Gew.-% enthält.

8. Verwendung nach einem der vorhergehenden Ansprüche, **dadurch gekennzeichnet, daß** die Zusammensetzung einen kosmetisch akzeptablen Träger umfaßt, der unter den organischen Lösungsmitteln, Wasser und/oder den Ölen ausgewählt ist.

9. Verwendung nach einem der vorhergehenden Ansprüche, **dadurch gekennzeichnet, daß** die Zusammensetzung ein filmbildendes Polymer enthält, das unter Nitrocellulose, Celluloseacetobutyrat, Butyralpolyvinylenen, Alkydharzen, Polyestern, Acrylverbindungen und Polyurethanen ausgewählt ist.

10. Verwendung nach Anspruch 9, **dadurch gekennzeichnet, daß** die Zusammensetzung einen Weichmacher enthält.

11. Verwendung nach einem der vorhergehenden Ansprüche, **dadurch gekennzeichnet, daß** die Zusammensetzung ein Nagellack oder eine Zusammensetzung zur Nagelpflege ist.

**Claims**

1. Use, in a cosmetic composition to be applied to the nail, of at least one monocarboxylic acid containing from 2 to 8 carbon atoms, and/or of one of its salts, as a hardening agent for the nails, the said acid containing no hydroxyl group.

2. Use according to Claim 1, **characterized in that** the monocarboxylic acid contains from 2 to 5 carbon atoms.

3. Use according to any one of the preceding claims, **characterized in that** the monocarboxylic acid is in free acid form.

4. Use according to one of the preceding claims, **characterized in that** the monocarboxylic acid is chosen from acetic acid, propionic acid, butyric acid, valeric acid, caproic acid, heptanoic acid or caprylic acid.

5. Use according to one of the preceding claims, **characterized in that** the monocarboxylic acid is acetic acid.

6. Use according to any one of the preceding claims, **characterized in that** the composition includes a monocarboxylic acid content of 0.01 % to 20 % by weight.

7. Use according to any one of the preceding claims, **characterized in that** the composition includes a monocarboxylic acid content of 0.5 % to 3 % by weight.

8. Use according to any one of the preceding claims, **characterized in that** the composition includes a cosmetically acceptable substrate chosen from organic solvents, water and/or oils.

9. Use according to any one of the preceding claims, **characterized in that** the composition includes a film-forming polymer chosen from nitrocellulose, cellulose acetobutyrate, polyvinylbutyral derivatives, alkyd resins, polyesters, acrylics and polyurethanes.

10. Use according to Claim 9, **characterized in that** the composition includes a plasticizer.

11. Use according to any one of the preceding claims, **characterized in that** the composition is a nail varnish or a composition for nail care.